(19) Europäisches Patentamt

European Patent Office

Office européen des brevets

(11) **EP 0 776 876 B1**

(12) **EUROPEAN PATENT SPECIFICATION**

(45) Date of publication and mention
of the grant of the patent:
**14.06.2000 Bulletin 2000/24**

(51) Int Cl.[7]: **C07C 15/02**, C07C 2/66,
C07B 61/00, B01J 29/70

(21) Application number: **95925993.8**

(22) Date of filing: **19.07.1995**

(86) International application number:
**PCT/JP95/01434**

(87) International publication number:
**WO 96/04225 (15.02.1996 Gazette 1996/08)**

(54) **METHOD OF LIQUID-PHASE ALKYLATION OF AROMATIC HYDROCARBON BY USING beta-ZEOLITE**

METHODE ZUR ALKYLIERUNG VON AROMATISCHEN KOHLENWASSERSTOFFEN IN FLÜSSIGER PHASE MIT beta-ZEOLITEN

PROCEDE D'ALKYLATION EN PHASE LIQUIDE D'HYDROCARBURES AROMATIQUES AU MOYEN DE LA beta-ZEOLITHE

(84) Designated Contracting States:
**DE**

(30) Priority: **03.08.1994 JP 18224294**

(43) Date of publication of application:
**04.06.1997 Bulletin 1997/23**

(73) Proprietor: **Asahi Kasei Kogyo Kabushiki Kaisha Osaka-shi, Osaka 530-8205 (JP)**

(72) Inventors:
• **ISHIDA, Hiroshi**
 **Okayama 710 (JP)**
• **TAKAMATSU, Yoshikazu**
 **Okayama 710-01 (JP)**

(74) Representative: **Strehl Schübel-Hopf & Partner Maximilianstrasse 54 80538 München (DE)**

(56) References cited:
 **EP-A- 0 432 814          JP-A- 3 181 424**
 **US-A- 5 227 558**

## Description

The present invention relates to a process for preparing alkylbenzenes useful as starting material of various types of polymers.

Background Art

Industrial processes for alkylation and transalkylation reactions of aromatic hydrocarbons are mostly carried out in the presence of a Friedel-Crafts catalyst such as aluminum chloride, silicontrifluoride, hydrofluoric acid, liquid and solid phosphoric acid, sulfuric acid or the like. Use of these catalysts, however, involves the risk of causing serious problems as such catalysts have corrosive action on the reaction apparatus. Substitution of such Friedel-Crafts catalysts with non-corrosive solid catalysts has been studied for many years, and various types of zeolite catalysts have been proposed as hopeful candidates for substitute catalysts.

The liquid phase or gas-liquid mixed phase reactions using these zeolite catalysts proceed under relatively mild conditions.

In US-A-4,169,111, US-A-4,185,040 and US-A-4,459,426,alkylation reaction of benzene with ethylene according to a fixed bed liquid phase reaction system using Y-type zeolite as catalyst is discussed.

JP-A-64-68329 describes transalkylation reaction of aromatic compounds according to a fixed bed liquid phase reaction system using a catalyst containing zeolite having a silica/alumina ratio of 40 or less, an a value of at least 400 and a constraint index of 5 to 9.

JP-A-2-96539 discusses alkylation reaction of benzene with an olefin with a carbon number of 8 to 16 according to a fixed bed liquid phase reaction system using zeolite as catalyst.

JP-A-2-174731 discloses alkylation reaction of benzene according to a fixed bed liquid phase reaction system using modified mordenite as catalyst.

JP-A-5-49652 proposes alkylation reaction of benzene according to a moving bed liquid phase reaction system using a zeolite molding as catalyst.

JP-A-61-161230 discloses alkylation reaction of benzene with olefins in a reaction distillation column having installed therein a fixed bed of Y-type zeolite.

EP-A-0 432 814 discloses liquid phase alkylation reaction of aromatic hydrocarbons with olefins using $\beta$-zeolite as catalyst. All of the Examples given therein, however, concern batchwise reaction.

JP-A-3-181424 states that in liquid phase alkylation or transalkylation reaction carried out under a condition where at least part of the reaction system is liquid phase in the presence of a $C_2$-$C_4$ olefin and an excess aromatic hydrocarbon (more than 4 times the molar quantity of olefin), $\beta$-zeolite allows formation of monoalkylation product in a higher yield over a far longer period of time than when using other types of zeolite catalysts, and also makes it possible to carry out the reaction with various types of reactor according to stirring reactor, fixed bed or fluidized bed system. According to the Examples of this reference, however, in view of the fact that the space velocity is about 5 on weight basis and the aromatic hydrocarbon to olefin molar ratio is 4 or above, the output of the alkylation product per unit weight of catalyst is very low. Also, in all of the Examples employing a fixed bed system in this reference, upflow packed bubble reaction system is used.

US-A-5,227,558 discloses preparation of ethylbenzene in a gaseous, liquid or gas-liquid mixed phase using $\beta$-zeolite which has been subjected to steaming. It is stated in this reference that upflow reaction system is preferred for the liquid phase or gas-liquid mixed phase reaction, and the upflow system is employed in the Examples.

In JP-A-4-187647, both alkylation reaction and alkyl transfer reaction are carried out in a liquid phase on a molecular sieve-based aromatic alkylating catalyst and an alkyl transfer catalyst. Up-flow flooded-bed reaction system is employed, and the liquid hourly space velocity (LHSV) is about 6 Hr[1] based on hydrocarbon feed. This reference states that it is unfavorable to carry out transalkylation reaction with Y zeolite as catalyst in a down-flow trickle bed reactor where gaseous phase exists substantially, because a rapid drop of activity is seen in the process of this reaction.

In alkylation reactions of aromatics, zeolite catalysts are of high utility as non-corrosive catalysts that can substitute the Friedel-Crafts catalysts, and many types of zeolite catalysts have been proposed. Zeolite catalysts, however, have some difficult problems that defy wide industrial use. For instance, under the normal industrial use conditions, zeolite catalysts are low in activity and also decline of their catalytic activity is fast, so that zeolite catalysts must be used in large quantities. Further, in stirring slurry process conducted for elevating activity of the catalyst, it is very difficult to separate the catalyst after reaction.

Use of ZSM-5 for gaseous phase alkylation of benzene with ethylene is known as an exceptional case. According to this method, the amount of the catalyst required for the reaction is very small, but this method involves the problems that the reaction temperature is as high as 430°C and that because of voluminous formation of by-product xylene, the cost for increasing the product purity in the refining step is high.

The present inventors, with a view to simplifying the reaction operation, have chosen a liquid phase reaction system under mild conditions and opted to carry out the reaction in a fixed bed for unnecessitating separation of the catalyst after reaction.

Some types of zeolite catalysts such as Y type and β type have been proposed for use in fixed bed liquid phase reactions, but most of these catalysts are low in activity, very low in productivity, and also rapidly reduced in activity in use, so that the catalyst needs to be used in large quantities for industrial practice of reaction.

Studies by the present inventors revealed that use of Y-type zeolite catalyst greatly lowers selectivity for alkylation of benzene nucleus (hereinafter referred to as nucleus alkylation) due to side chain alkylation, formation of by-product diphenylalkanes and other causes with progress of the reaction.

It has been reported recently that in alkylation reaction of aromatic hydrocarbons according to a fixed-bed upflow packed bubble reaction system, β-zeolite shows high selectivity as compared with other types of zeolite catalyst and is also very limited in decline of activity in use. Even in this method, however, the aromatic to olefin molar ratio is 4 or above and the weight hourly space velocity (WHSV), which is a measure of throughput of aromatic hydrocarbon per unit weight of catalyst, is as low as about 5 $Hr^{-1}$. Therefore, even if an olefin could be entirely converted by use of this catalyst, the yield of alkylation product is very low. It has also been reported that even if the olefin feed is increased, decline of catalytic activity is rapid under a condition which allows the unreacted olefin to remain in the system.

The above problems remained to be solved for realizing effective industrial utilization of the conventional zeolite catalysts.

Disclosure of Invention

Strenuous studies by the present inventors on the subject matter have led to the discovery that in liquid phase alkylation of aromatic hydrocarbons using a $C_2$-$C_4$ olefin in a fixed bed reactor packed with a β-zeolite catalyst and maintained at 100-300°C, when a cocurrent down flow trickle-bed reaction system is employed, it is possible to attain a very high yield and productivity, nucleus alkylation reaction of aromatic hydrocarbons can be performed with high selectivity, and also decline of catalyst activity in use is restrained. The present invention has been attained on the basis of this discovery.

The present invention provides a liquid phase alkylation process for aromatic hydrocarbons which comprises reacting an aromatic hydrocarbon with a $C_2$-$C_4$ olefin (alkylating agent) in a fixed bed reactor packed with a β-zeolite catalyst, in the trickle flow region according to a cocurrent down flow reaction system at 100 to 300°C.

Brief Description of the Drawing

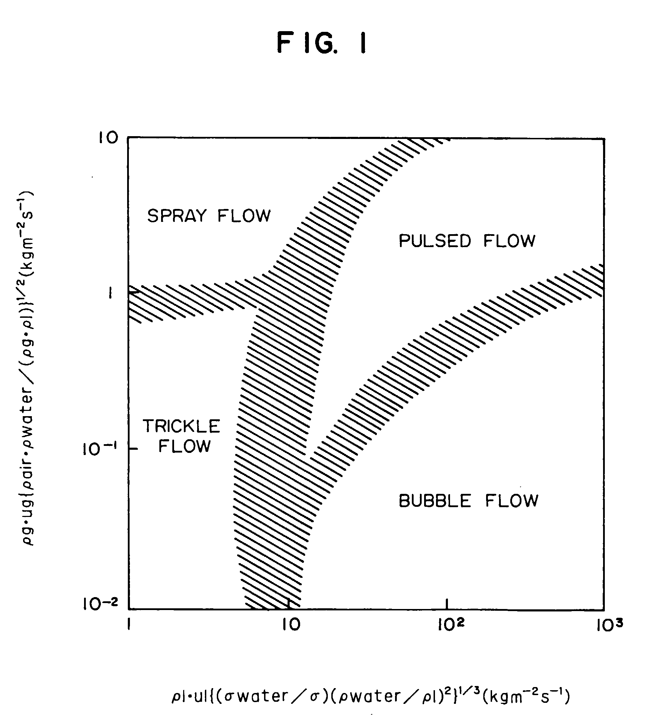

FIG. 1 is a graphic illustration of the flow zones in a cocurrent down flow reaction system.

Best Mode for Carrying Out the Invention

The catalyst used in the present invention is β-zeolite. β-zeolite is a known synthetic crystalline aluminosilicate described for the first time in US-A-3,308,069, and identified by its characteristic X-ray diffraction image as described in the specification of this patent. The reflection d values of X-ray diffraction of β-zeolite are shown in Table 1.

Table 1:

| Reflection d values of β-zeolite | | |
|---|---|---|
| 11.4 | ± | 0.2 Å |
| 7.4 | ± | 0.2 |
| 6.7 | ± | 0.2 |
| 4.25 | ± | 0.1 |
| 3.97 | ± | 0.1 |
| 3.0 | ± | 0.1 |
| 2.2 | ± | 0.1 |

The $SiO_2/Al_2O_3$ ratio of β-zeolite used in the present invention is in a range of 5 to 100, preferably 10 to 60, more preferably 15 to 40.

β-zeolite used in the present invention is of acid type, obtained by replacing sodium ions with hydrogen ions and/or polyvalent cations by ion exchange method. If β-zeolite originally has a sufficiently high [organic cation/sodium ion] ratio, it suffices to calcine the zeolite with no ion exchange.

For obtaining a higher catalytic activity, it is desirable to turn the zeolite into a hydrogen ion type by ion exchange method. The synthesized β-zeolite is calcined to remove the organic matter and then subjected to ion exchange. Conversion to hydrogen ion type is usually accomplished by stirring the calcined β-zeolite in a dilute nitric acid aqueous solution. The thus treated β-zeolite is dried to reduce the water content to less than 10 parts by weight.

The β-zeolite catalyst to be packed in the reactor is a molding. The molding may be composed of pure zeolite alone or may contain an inorganic oxide(s) such as alumina, silica, silica/alumina or natural clay as binder. Suitable methods well known in the art, such as tableting and extrusion molding, may be used for obtaining the molding. The shape of the catalyst molding is usually cylindrical, but it may be spherical, plate-like, hollow-cylindrical or such.

In the present invention, the liquid flow (residence time distribution) in the reactor is preferably approximated to the ideal piston flow for obtaining a high catalytic activity. For this reason, the particle size of the catalyst (molding) used in the present invention is preferably defined to be less than 0.2 time the inner diameter of the reactor. In the present specification, the particle size of the catalyst means the diameter of a sphere having a volume equal to that of the corresponding catalyst particle.

The reactor used in the present invention is a fixed bed reactor and usually cylindrical in shape. The inner diameter of the reactor is decided in consideration of the heat of reaction and the heat eliminated but it is usually in a range of 1.27 to 5.08 cm (0.5 to 2 inches).

In the present invention, the reaction is carried out according to a cocurrent down flow system, specifically in a trickle flow region where the gaseous material takes a continuous phase while the liquid material takes a disperse phase.

In this reaction system, the liquid drips down along the outer surface of the solid catalyst. Part of the liquid is caught in small interstices in the solid catalyst. Either way, the liquid is interspersed in clusters, forming a disperse phase. On the other hand, the gaseous body surrounds the solid catalyst and the liquid to form a continuous phase. The flow regions in the cocurrent down flow reaction system are shown in FIG. 1. This diagram was drawn up based on the data obtained from the air-water system. For other systems, the similar charts of flow regions can be prepared by taking into consideration the correction terms based on the difference of physical properties. The hatched portion indicates the boundary area. (See SHOKUBAIKOZA (Lectures on Catalysts) Vol. 6, 2nd Ed., compiled by Shokubai Gakkai).

In the present invention, "trickle flow region" refers to a flow region in a cocurrent down flow system which meets the following conditions:

$$\rho l \cdot ul \cdot \{(\sigma water/\sigma)(\rho water/\rho l)^2\}^{1/3} \ (kgm^{-2}s^{-1}) \leq 10$$

and

$$\rho g \cdot ug \cdot \{\rho air \cdot \rho water/(\rho g \cdot \rho l)\}^{\frac{1}{2}} \ (kgm^{-2}s^{-1}) \leq 1$$

In the above formulae, $\rho l$, $\rho g$, $\rho air$ and $\rho water$ ($kgm^{-3}$) denote liquid density of aromatic hydrocarbon, gas density of olefin, gas density of air and liquid density of water, respectively, $\sigma$ and $\sigma water$ ($Nm^{-1}$) denote surface tensions of aromatic hydrocarbon and water, respectively, and $ul$ and $ug$ ($ms^{-1}$) denote superficial velocities in a column of aromatic hydrocarbon and olefin, respectively.

The reaction is carried out in a flow region meeting the above conditions, preferably in the trickle flow region and the boundary area indicated by hatching in FIG. 1, more preferably in a region which is obviously recognized as trickle flow region.

It has hitherto been considered undesirable to carry out an alkylation reaction of an aromatic hydrocarbon with an olefin in the presence of a zeolite catalyst according to such a cocurrent down flow system because the catalytic activity is excessively reduced by catalyst-olefin contact. However, according to the present invention, it was found quite surprisingly that in carrying out an alkylation reaction of an aromatic hydrocarbon with an olefin according to a cocurrent down flow system using β-zeolite as catalyst, when this reaction is conducted in the trickle flow region where the gaseous material constitutes a continuous phase while the liquid material forms a disperse phase, the nucleus alkylation of the aromatic hydrocarbon can advance with high selectivity, with the catalytic activity being kept from being reduced for a long time, and further the catalyst can maintain high activity and hence the productivity is markedly enhanced as compared with other fixed bed reaction systems, for example, an upflow packed bubble reaction system.

The aromatic hydrocarbons usable in the present invention include benzene, toluene, ethyl-benzene and xylene, of which benzene is preferred. These aromatic hydrocarbons may be used either singly or as a mixture.

The $C_2$-$C_4$ olefins usable as alkylating agent in the present invention are those having 2 to 4 carbon atoms in the molecule, which include ethylene, propylene, butene-1, trans-butene-2 and cis-butene-2. These olefins may be used either singly or as a mixture. Ethylene and propylene are preferred, the former being more preferred.

The material feed rate in the present invention is in the above-defined range for the trickle flow region, but for attaining a high productivity, it is preferably adjusted so that the weight hourly space velocity (WHSV) based on the weight of β-zeolite will fall in a range of 100 or less, preferably 60 or less. A $C_2$-$C_4$ olefin used as alkylating agent is supplied at a rate that conforms to the rate of reaction decided by the reaction conditions, namely olefine consumption rate, and that allows maintenance of internal pressure of the system. For instance, the reaction is carried out employing a constant-pressure gas phase charge system in which the consumed reactants are supplied constantly.

Reaction temperature in the present invention is in a range of 100 to 300°C, preferably 120 to 230°C, more preferably 150 to 200°C.

Reaction pressure in the present invention differs depending on the material and reaction temperature, but it may be a pressure sufficient to maintain the liquid phase, usually in a range of 1 to 5 MPa (10 to 50 atm).

The present invention is explained below with reference to the examples but should not be constructed to be limited thereto so far as the invention is not out of the gist thereof.

Example 1

(1) Synthesis of β-zeolite

To a mixture of 160 g of an aqueous 10% tetraethylammonium hydroxide solution, 140 g of water, 4.2 g of sodium hydroxide and 9.5 g of sodium aluminate $NaAlO_2$, 70.5 g of fused silica NIPSEAL (NV-3 produced by Nippon Silica Industries Co., Ltd.) and 7 g of β-zeolite (as seed crystal) were added and the obtained mixture was stirred at 5,000 rpm for 30 minutes by a homogenizer. The resulting mixture was put into a 500 ml autoclave and allowed to stand at 155°C for 8 days without stirring. As a result, a large amount of crystalline material was produced. This material was filtered and the crystals that were left were washed with water and dried for a whole day at 120°C to give 68 g of crystalline powder. This crystalline powder was gradually heated to 350-550°C and finally calcined at 550°C for 6 hours. X-ray diffraction of the calcined powder identified it as β-zeolite.

(2) Hydrogen ion exchange of β-zeolite

Fifty grams of calcined β-zeolite was added to 450 g of a 0.15N aqueous nitric acid solution cooled to 3°C and the mixture was stirred under ice cooling at 3°C for 2 hours to effect ion exchange. Thereafter, the resulting product was filtered, washed with water and then dried at 120°C. Compositional analysis of the obtained hydrogen ion type β-zeolite was made by an X-ray microanalyzer (EPMA). The silica/alumina ratio was 21.

(3) Evaluation of reaction

The above hydrogen ion type β-zeolite was compression molded by a tableting machine, then pulverized and classified to obtain 2.5 g of 8- to 12-mesh molded catalyst. This molded catalyst was packed in a stainless steel tubular reactor having a heat transfer jacket having an inner diameter of 10.5 mm and a length of 500 mm while uniformly diluting the molded catalyst with 6 times as much volume of 20- to 30-mesh quartz sand. A 3 mm$\phi$ stainless steel Dixon packing was provided as preheating layer on top of the catalyst layer. At the outlet of the reactor was provided a liquid receiving tank which has been equalized to the reactor in pressure. Ethylene gas was charged into the system at room temperature by a pressure regulating valve until the system had a constant pressure of 18.5 kg/cm$^2$G. In this system, ethylene is supplied in an amount sufficient to make up for the consumption in the reaction. Benzene was supplied at a rate of 130 ml/hr from the top of the reactor. Weight hourly space velocity (WHSV) was 45.8 g-BZ/g-cat/hr. It is also apparent from the following data that the flow region where the reaction took place in this Example was the trickle flow region:

$$\rho l \cdot ul \cdot \{(\sigma water/\sigma)(\rho water/\rho l)^2\}^{1/3} \ (kgm^{-2}s^{-1}) = 0.75$$

$$\rho g \cdot ug \cdot \{\rho air \cdot \rho water/(\rho g \cdot \rho l)\}^{\frac{1}{2}} \ (kgm^{-2}s^{-1}) = 0.06$$

Thereafter, the heat transfer medium was circulated in the reactor jacket. The heat transfer medium was heated gradually till temperature in the catalyst layer reached 190°C. Ever after 40 hours from the start of the reaction, the heat transfer medium temperature and the maximum temperature reached in the catalyst layer became constant at 170°C and 192°C, respectively. The reaction was continued under these conditions for 300 hours. Periodical analysis of the samples of the produced liquid was made by gas chromatography. The obtained reaction results are shown in Table 2.

Table 2:

| Results of ethylation reaction of benzene with β-zeolite catalyst (Fixed trickle bed reaction system) | | | | | | |
|---|---|---|---|---|---|---|
| Reaction time (Hr) | | 5 | 50 | 100 | 300 |
| Produced liquid composition wt% | Benzene | 54.22 | 70.94 | 75.53 | 75.57 |
| | Ethylbenzene | 35.20 | 24.66 | 21.26 | 21.30 |
| | Diethylbenzene | 8.85 | 3.99 | 2.92 | 2.86 |
| | Triethylbenzene | 1.24 | 0.36 | 0.23 | 0.22 |
| | Tetraethylbenzene | 0.07 | 0.02 | 0.01 | 0.01 |
| | s-butylbenzene | 0.40 | 0.04 | 0.04 | 0.03 |
| | Diphenylethanes | 0.013 | 0.004 | 0.003 | 0.002 |
| | Others | 0.00 | 0.00 | 0.00 | 0.00 |
| Ethyl group/benzene ring molar ratio | | 0.448 | 0.255 | 0.209 | 0.208 |
| EB~E4B selectivity [1] | | 99.24 | 99.86 | 99.86 | 99.86 |

[1] Selectivity of nucleus ethylation product (on converted benzene basis) mol%

The catalyst activity lowered gradually till 55 hours from the start of the reaction, but after 60 hours, a steady level of activity was maintained, and thereafter till the end of the reaction (300 hours), no worsening of the reaction result was observed. Reaction rate in the steady state of activity was 0.123 mol-ethyl group/g-cat/hr. On the ethylbenzene basis, the reaction showed a productivity of 13.0 g-EB/g-cat/hr. (Polyethylbenzene can be entirely converted to ethylbenzene by transalkylation). Further, selectivity of the nucleus ethylation product on the converted benzene basis was as high as 99.86%, and its reaction performance was maintained through 300 hours of the reaction.

From this Example and Comparative Examples 1 and 2 described later, it is seen that in the fixed bed liquid phase alkylation reaction of aromatic compounds using β-zeolite as catalyst, it is possible to attain a very high activity (productivity) and selectivity and to keep the catalytic activity from lowering for a long time by employing a fixed bed cocurrent down flow reaction system where the reaction is carried out in the trickle flow region.

Example 2

β-zeolite (C-806β, tetraethylammonium type, produced by PQ Corp.) was calcined at 550°C, then converted to hydrogen ion type and molded according to the procedure of Example 1. Compositional anlaysis of the obtained hydrogen ion type β-zeolite was made by an X-ray microanalyzer (EPMA). The silica/alumina ratio was 38.

Except for using the thus obtained hydrogen ion type β-zeolite, the same reaction as in Example 1 was carried out for 100 hours. Forty hours were taken till a steady state of activity was reached, but thereafter no reduction of reaction performance was observed. The reaction results are shown in Table 3.

Table 3:

| Results of ethylation reaction of benzene with β-zeolite catalyst (Fixed trickle bed reaction system) | | | |
|---|---|---|---|
| Reaction time (Hr) | | 50 | 100 |
| Produced liquid composition wt% | Benzene | 77.02 | 77.00 |
| | Ethylbenzene | 20.15 | 20.16 |
| | Diethylbenzene | 2.60 | 2.60 |
| | Triethylbenzene | 0.19 | 0.19 |
| | Tetraethylbenzene | 0.01 | 0.01 |
| | s-butylbenzene | 0.04 | 0.04 |
| | Diphenylethanes | 0.003 | 0.002 |
| | Others | 0.00 | 0.00 |

Table 3:   (continued)

| Results of ethylation reaction of benzene with β-zeolite catalyst (Fixed trickle bed reaction system) | | |
|---|---|---|
| Reaction time (Hr) | 50 | 100 |
| Ethyl group/benzene ring molar ratio | 0.194 | 0.195 |
| EB∼E4B selectivity | 99.86 | 99.85 |

Reaction rate in the steady state was 0.114 mol-ethyl group/g-cat/hr, and productivity on the ethylbenzene basis was 12.1 g-EB/g-cat/hr. Also, selectivity of the nucleus ethylation product on the converted benzene basis was as high as 99.86%.

Example 3

β-zeolite (C-806β, tetraethylammonium type, produced by PQ Corp.) was calcined at 550°C and then converted to hydrogen ion type according to the procedure of Example 1 to obtain hydrogen ion type β-zeolite. Compositional analysis of the obtained hydrogen ion type β-zeolite was made by an X-ray microanalyzer (EPMA). The silica/alumina ratio was 38.
The thus obtained hydrogen ion type β-zeolite was molded into 3 mmφ x 2 mmL pellets by a tableting machine.
Ninety grams of the molded catalyst was packed, without dilution, in a stainless steel tubular reactor with a heat transfer jacket having an inner diameter of 22.1 mm and a length of 1,000 mm. A 3 mmφ stainless steel Dixon packing was packed as a preheating layer on the top of the catalyst layer. At room temperature, benzene was supplied at a rate of 3,170 ml/hr from the top of the tubular reactor while ethylene was fed at a rate of 4.5 NL/min by a mass flowmeter. Pressure in the system was adjusted to stay constant at 18.5 kg/cm$^2$G by a pressure regulating valve provided at the gas phase outlet of the gas-liquid separator which was set at the reactor exit. Weight hourly space velocity (WHSV) based on packed β-zeolite was 31 g-BZ/g-cat/hr. Also, as seen from the following data, the flow region in this Example was obviously a trickle bed region:

$$\rho l \cdot ul \cdot \{(\sigma water/\sigma)(\rho water/\rho l)^2\}^{1/3} \ (kgm^{-2}s^{-1}) = 4.1$$

$$\rho g \cdot ug \cdot \{\rho air \cdot \rho water/(\rho g \cdot \rho l)\}^{½} \ (kgm^{-2}s^{-1}) = 0.5$$

Thereafter, the heat transfer medium was circulated in the reactor jacket and heated gradually. After 20 hours, heat transfer medium temperature become constant at 120°C while internal temperature of the catalyst layer reached the maximum temperature of 196°C at the lower part of the layer and stayed constant there. The reaction was conducted for 60 hours. After 15 hours, almost no reduction of reaction performance was observed. The reaction results are shown in Table 4.

Table 4:

| Results of ethylation reaction of benzene with β-zeolite catalyst (Fixed trickle bed reaction system) | | | | |
|---|---|---|---|---|
| Reaction time (Hr) | | 10 | 30 | 60 |
| Produced liquid composition wt% | Benzene | 63.68 | 66.64 | 66.61 |
| | Ethylbenzene | 29.57 | 27.60 | 27.56 |
| | Diethylbenzene | 5.93 | 5.09 | 5.16 |
| | Triethylbenzene | 0.69 | 0.56 | 0.57 |
| | Tetraethylbenzene | 0.04 | 0.03 | 0.03 |
| | s-butylbenzene | 0.06 | 0.06 | 0.05 |
| | Diphenylethanes | 0.01 | 0.01 | 0.01 |
| | Others | 0.02 | 0.01 | 0.00 |
| Ethyl group/benzene ring molar ratio | | 0.334 | 0.301 | 0.302 |

Table 4:   (continued)

| Results of ethylation reaction of benzene with β-zeolite catalyst (Fixed trickle bed reaction system) | | | |
|---|---|---|---|
| Reaction time (Hr) | 10 | 30 | 60 |
| EB~E4B selectivity | 99.82 | 99.82 | 99.83 |

Reaction rate in the steady state in the present Example was 0.12 mol-ethyl group/g-cat/hr, and productivity on the ethylbenzene basis was 12.7 g-EB/g-cat/hr. Also, selectivity of nucleus ethylation product on the converted benzene basis was as high as 99.83%.

From the results of this Example and those of Example 4 and Comparative Examples 3 and 4 described below, it is seen that in the fixed bed liquid phase alkylation reaction of aromatic compounds using β-zeolite as catalyst, it is possible to attain a very high activity (productivity) and selectivity and to restrain decline of activity by employing a cocurrent down flow reaction system where the reaction is substantially carried out in the trickle flow region.

Example 4

The same reaction procedure was followed as in Example 3, except that a stainless steel tubular reactor with a heat transfer jacket having an inner diameter of 41.2 mm and a length of 5,000 mm, was used and the reactor was packed with 500 g of pelletized catalyst, and that benzene was supplied at a rate of 20 L/hr while feeding ethylene at a rate of 30 NL/min. The weight hourly space velocity (WHSV) relative to the packed β-zeolite was 35 g-BZ/g-cat/hr. The following data confirm that the flow region in this Example is a trickle flow region:

$$\rho l \cdot ul \cdot \{(\rho water/\rho)(\rho water \cdot \rho l)^2\}^{1/3} \ (kgm^{-2}s^{-1}) = 7.5$$

$$\rho g \cdot ug \cdot \{\rho air \cdot \rho water/(\rho g \cdot \rho l)\}^{\frac{1}{2}} \ (kgm^{-2}s^{-1}) = 1.0$$

After 10 hours from the start of the reaction, heat transfer medium temperature became constant at 104°C while internal temperature of the catalyst layer reached and stayed at the maximum temperature of 200°C at the lower part of the layer. The reaction was conducted for 30 hours. After 15 hours, no drop of reaction performance was observed. The reaction results are shown in Table 5.

Table 5:

| Results of ethylation reaction benzene with β-zeolite catalyst (Fixed trickle bed reaction system) | | | | |
|---|---|---|---|---|
| Reaction time (Hr) | | 10 | 20 | 30 |
| Produced liquid composition wt% | Benzene | 64.91 | 69.00 | 68.92 |
| | Ethylbenzene | 28.79 | 25.91 | 26.01 |
| | Diethylbenzene | 5.56 | 4.53 | 4.51 |
| | Triethylbenzene | 0.63 | 0.47 | 0.46 |
| | Tetraethylbenzene | 0.03 | 0.02 | 0.02 |
| | s-butylbenzene | 0.06 | 0.05 | 0.05 |
| | Diphenylethanes | 0.02 | 0.01 | 0.01 |
| | Others | 0.02 | 0.01 | 0.00 |
| Ethyl group/benzene ring molar ratio | | 0.320 | 0.276 | 0.277 |
| EB~E4B selectivity | | 99.82 | 99.83 | 99.83 |

In this Example, reaction rate was 0.124 mol-ethyl group/g-cat/hr, and productivity based on the ethylbenzene basis was 13.1 g-EG/g-cat/hr. Further, selectivity of nucleus ethylation product on the converted benzene basis was as high as 99.83%.

Example 5

The same reaction procedure was carried out as in Example 1 except that propylene was used as alkylating olefin and the pressure in the system was adjusted to 10 kg/cm$^2$G and that the reaction temperature was controlled so that the heat transfer medium temperature would become constant at 150°C and the internal temperature of the catalyst layer at 170°C. The reaction was continued for 24 hours, during which no decline of reaction performance was observed. The reaction results are shown in Table 6.

Table 6:

| Results of isopropylation reaction of benzene with β-zeolite catalyst (Fixed trickle bed reaction system) | | | |
|---|---|---|---|
| Reaction time (Hr) | | 5 | 24 |
| Produced liquid composition wt% | Benzene | 62.43 | 62.44 |
| | n-Propylbenzene | 0.00 | 0.00 |
| | Cumene | 34.94 | 34.97 |
| | Diisopropylbenzene | 2.23 | 2.20 |
| | Others | 0.40 | 0.39 |
| Isopropyl group/benzene ring molar ratio | | 0.29 | 0.29 |

Reaction rate was 0.17 mol-isopropyl group/g-cat/hr, and productivity calculated on the cumene basis was 20.4 g-cumene/ g-cat/hr. Selectivity of the nucleus propylation product on the converted benzene basis was also as high as 98.8%.

Comparative Example 1

In the same way as in Example 1, 2.5 g of 8-to 12-mesh molded catalyst of hydrogen ion type β-zeolite was obtained, which was packed in a fixed bed reactor (10.5 mm in inner diameter and 500 mm in length; stainless steel tubular reactor with heat transfer jacket), as used in Example 1, and subjected to liquid phase alkylation reaction according to the upflow packed bubble reaction system. A pressure dwelling valve was provided at the outlet at the top of the reactor and benzene was supplied from the bottom of the reactor at a rate of 13.1 ml/hr. The weight hourly space velocity (WHSV) was 4.6 g-BZ/g-cat/hr. Effluent was discharged out through the pressure dwelling valve by which the internal pressure of the system was set at 20 kg/cm$^2$G. A 170°C heat transfer medium was circulated in the reactor jacket. After confirming perfect benzene replacement of the reactor atmosphere, ethylene gas was supplied from the reactor bottom at a rate of 0.473 NL/hr. The benzene/ethylene molar ratio was 7. After 30 hours of reaction under the same conditions, the benzene feed rate was raised to 130 ml/hr (WHSV = 45.8 g-BZ/g-cat/hr) and the ethylene feed rate to 13.14 NL/hr (benzene/ethylene molar ratio = 2.5). That is, the reaction was conducted at the same WHSV as in Example 1. After said change of conditions, the reaction was continued for 6 hours. The reaction results are shown in Table 7.

Table 7:

| Results of ethylation reaction of benzene with β-zeolite catalyst (Fixed bed upflow packed bubble system) | | | | |
|---|---|---|---|---|
| Reaction time (Hr) | 10 | 28 | 32 | 37 |
| WHSV (Hr$^{-1}$) | 4.6 | | 45.8 | |
| Benzene/ethylene | 7 | | 2.5 | |

Table 7: (continued)

| Results of ethylation reaction of benzene with β-zeolite catalyst (Fixed bed upflow packed bubble system) | | | | | |
|---|---|---|---|---|---|
| Reaction time (Hr) | | 10 | 28 | 32 | 37 |
| Produced liquid composition wt% | Benzene | 82.42 | 82.49 | 94.92 | 96.64 |
| | Ethylbenzene | 16.01 | 15.98 | 4.82 | 3.28 |
| | Diethylbenzene | 1.39 | 1.37 | 0.12 | 0.06 |
| | Triethylbenzene | 0.05 | 0.05 | 0.01 | 0.00 |
| | Tetraethylbenzene | 0.00 | 0.00 | 0.00 | 0.00 |
| | s-butylbenzene | 0.09 | 0.08 | 0.13 | 0.01 |
| | Diphenylethanes | 0.00 | 0.00 | 0.00 | 0.00 |
| | Others | 0.04 | 0.03 | 0.01 | 0.01 |
| Ethyl group/benzene ring molar ratio | | 0.143 | 0.142 | 0.0374 | 0.0251 |
| EB~E4B selectivity | | 99.01 | 99.45 | 97.84 | 99.34 |

As seen from the above Comparative Example, in a liquid phase alkylation according to the upflow packed bubble reaction system using β-zeolite, when WHSV = 4.6 and benzene/ethylene = 7, although no drop of activity was observed, the reaction rate stayed low at 0.0085 mol-ethyl group/g-cat/hr while the productivity on the ethylbenzene basis was as low as 0.9 g-EB/g-cat/hr, which is only about 7% or less of the productivity in the steady state in Example 1.

Even when the reaction was carried out under the conditions of WHSV = 45.8 and benzene/ethylene = 2.5, that is, under the same throughput conditions as in Example 1, the reaction was still low at 0.022 mol-ethyl group/g-cat/hr and the productivity on the ethylbenzene basis was no better than 2.3 g-EB/g-cat/hr, which is about 20% or less of the steady-state productivity according to the system of Example 1. Further, decline of reactivity was noted in only 5 to 6 hours of reaction after change of reaction conditions.

Comparative Example 2

The same reaction procedure was followed as in Example 1, except for use of the catalyst obtained by calcining steam-stabilized Y-type zeolite (LINDE LZ-Y82) at 450°C for 6 hours, compression molding it and crashing and classifying the molding to 8- to 12-mesh particle size. Although heat transfer medium circulation temperature was the same (170°C) as in Example 1, the maximum temperature reached in the catalyst layer was 177°C. The reaction was conducted for 12 hours. The reaction results are shown in Table 8.

Table 8:

| Results of ethylation reaction of benzene with Y-zeolite catalyst (Fixed trickle bed reaction system) | | | |
|---|---|---|---|
| Reaction time (Hr) | | 6 | 10 |
| Produced liquid composition wt% | Benzene | 92.49 | 94.53 |
| | Ethylbenzene | 5.87 | 4.26 |
| | Diethylbenzene | 0.59 | 0.28 |
| | Triethylbenzene | 0.10 | 0.06 |
| | Tetraethylbenzene | 0.02 | 0.01 |
| | s-butylbenzene | 0.30 | 0.28 |
| | Diphenylethanes | 0.05 | 0.04 |
| | Others | 0.57 | 0.54 |
| Ethyl group/benzene ring molar ratio | | 0.064 | 0.046 |
| EB~E4B selectivity | | 89.57 | 86.69 |

As seen from the above Comparative Example, in a liquid phase alkylation process according to the fixed trickle bed reaction system using Y-zeolite, the reaction rate was as low as 0.037 mol-ethyl group/g-cat/hr. On the ethylbenzene basis, productivity was only 4.0 g-EB/g-cat/hr, which is as low as 30% of the steady-state productivity in Example 1 using β-zeolite. Selectivity of nucleus ethylation product on the converted benzene basis was also as low as 89.6%.

Comparative Example 3

The same reaction procedure was repeated as in Example 3, except that the ethylene feed rate was set at 55 NL/min. The weight hourly space velocity (WHSV) relative to the packed β-zeolite was 31 g-BZ/g-cat/hr, as in Example 3. However, as seen from the following data, the flow region in this Comparative Example obviously deviated from the trickle flow region in the present invention, and it coincided with the spray flow region where the liquid is formed into droplets because of swift gas flow:

$$\rho l \cdot ul \cdot \{(\sigma water/\sigma)(\rho water/\rho l)^2\}^{1/3} \ (kgm^{-2}s^{-1}) = 4.1$$

$$\rho g \cdot ug \cdot \{\rho air \cdot \rho water/(\rho g \cdot \rho l)\}^{\frac{1}{2}} \ (kgm^{-2}s^{-1}) = 6.2$$

When the temperature of the heat transfer medium was raised gradually from the start of the reaction, an abnormal rise of temperature took place in the middle portion of the catalyst layer, elevating the internal temperature to 350°C to render the reaction uncontrollable. The reaction was stopped at this point.
Since the recovered catalyst after the completion of the reaction was covered with coke, it was judged that the catalyst had been perfectly deactivated.
As seen from the above Comparative Example, in a fixed bed liquid phase alkylation reaction of aromatic compounds using β-zeolite as catalyst, when the reaction is carried out in the spray flow region, which is a flow region deviating from the trickle flow region, in the cocurrent down flow reaction system, it is impossible to maintain the liquid phase reaction due to runaway of the reaction and the catalyst is deactivated instantaneously.

Comparative Example 4

The same reaction procedure was followed as in Example 4, except that the amount of the β-zeolite catalyst molding packed in the reactor was 2,130 g, and that the benzene feed rate was 85 L/hr. The weight hourly space velocity (WHSV) relative to the packed β-zeolite was 35 g-BZ/g-cat/hr, as in Example 4. However, as recognized from the following data, the flow region in this Comparative Example obviously deviated from the trickle flow region envisaged in the present invention and was synonymous with the pulsed flow region where the liquid phase is a continuous phase and the bubbles flowing a dispersed state:

$$\rho l \cdot ul \cdot \{(\sigma water/\sigma)(\rho water/\rho l)^2\}^{1/3} \ (kgm^{-2}s^{-1}) = 31.7$$

$$\rho g \cdot ug \cdot \{\rho air \cdot \rho water/(\rho g \cdot \rho l)\}^{\frac{1}{2}} \ (kgm^{-2}s^{-1}) = 1.0$$

Temperature of the heat transfer medium was gradually raised from the start of the reaction, and the medium temperature was set so that the maximum temperature in the catalyst layer would become 200°C as in Example 4. The produced liquid after 3 hours from the start of the reaction had the following composition:

| Benzene | 94.65 wt% |
|---|---|
| Ethylbenzene | 5.13 |
| Diethylbenzene | 0.19 |
| Butylbenzene | 0.02 |
| Others | 0.01 |
| Ethyl group/benzene ring molar ratio: 0.041 | |

Reaction rate in this Comparative Example was 0.018 mol-ethyl group/g-cat/hr, and productivity on the ethyl-

benzene basis was as low as 1.96 g-EB/g-cat/hr, indicating very low catalyst activity in this reaction system as compared with Example 4.

It can be seen from the above Comparative Example that in a fixed bed liquid phase alkylation reaction of aromatic compounds using β-zeolite catalyst, when the reaction is carried out in the bubble flow region, which is a flow region deviating from the trickle flow region adopted in the present invention, according to the cocurrent down flow reaction system, catalyst activity is lowered excessively.

Comparative Example 5

The same reaction procedure was carried out as in Example 4, except for use of a stainless steel tubular reactor with a heat transfer jacket having an inner diameter of 22.1 mm and a length of 5,000 mm. The weight hourly space velocity (WHSV) relative to the packed β-zeolite was 35 g-BZ/g-cat/hr, as in Example 4. However, as noted from the following data, the flow region in this Comparative Example obviously deviated from the trickle flow region in the present invention and was synonymous with the pulsed or slug flow region where gas flow and liquid flow take place alternately:

$$\rho l \cdot ul \cdot \{(\sigma water/\sigma)(\rho water/\rho l)^2\}^{1/3} \ (kgm^{-2}s^{-1}) = 25.9$$

$$\rho g \cdot ug \cdot \{\rho air \cdot \rho water/(\rho g \cdot \rho l)\}^{\frac{1}{2}} \ (kgm^{-2}s^{-1}) = 3.4$$

Temperature of the heat transfer medium was raised gradually from the start of the reaction and controlled so that the maximal temperature in the catalyst layer would become 200°C, as in Example 4. The produced liquid on passage of 3 hours from the start of the reaction had the following composition:

| Benzene | 93.68 wt% |
|---|---|
| Ethylbenzene | 6.05 |
| Diethylbenzene | 0.23 |
| Butylbenzene | 0.02 |
| Others | 0.02 |
| Ethyl group/benzene ring molar ratio: 0.048 | |

Reaction rate in this Comparative Example was 0.022 mol-ethyl group/g-cat/hr, and productivity on the ethylbenzene basis was 2.30 g-EB/g-cat/hr, indicating very low catalyst activity as compared with Example 4.

As seen from the above Comparative Example, in a fixed bed liquid phase alkylation reaction of aromatic compounds using β-zeolite as catalyst, when the reaction is carried out in the pulsed flow region, which is a flow region deviating from the trickle flow region of this invention, in the cocurrent down flow reaction system, catalyst activity is lowered excessively.

Industrial Applicability

According to the present invention, by carrying out liquid phase alkylation reaction of aromatic hydrocarbons in the trickle flow region in a cocurrent down flow reaction system in a fixed bed reactor using a β-zeolite catalyst, it is possible to maintain high catalyst activity (high productivity) and to perform nucleus alkylation of aromatic hydrocarbons with high selectivity. It is also possible to maintain catalyst activity for a long time. These effects are beneficial to the industrial utilization of the present invention.

**Claims**

1.  A liquid phase alkylation process for aromatic hydrocarbons, which comprises reacting an aromatic hydrocarbon with a $C_2$-$C_4$ olefin in a liquid phase in a trickle flow region in a fixed bed reactor packed with a β-zeolite catalyst according to a cocurrent down flow reaction system at 100 to 300°C, said trickle flow region being specified by meeting the following conditions:

$$\rho l \cdot ul \cdot \{(\sigma water/\sigma)(\rho water/\rho l)^2\}^{1/3} \ (kgm^{-2}s^{-1}) \leq 10$$

$$\rho g \cdot ug \cdot \{\rho air \cdot \rho water/(\rho g \cdot \rho l)\}^{\frac{1}{2}} \ (kgm^{-2}s^{-1}) \leq 1$$

wherein $\rho l$, $\rho g$, $\rho air$ and $\rho water$ ($kgm^{-3}$) denote liquid density of the aromatic hydrocarbon, gas density of the $C_2$-$C_4$ olefin, gas density of air and liquid density of water, respectively, $\sigma$ and $\sigma water$ ($Nm^{-1}$) denote surface tensions of the aromatic hydrocarbon and water, respectively, and $ul$ and $ug$ ($ms^{-1}$) denote superficial velocities in a column of the aromatic hydrocarbon and $C_2$-$C_4$ olefin, respectively.

2. The process according to Claim 1, wherein the aromatic hydrocarbon is at least one member selected from the group consisting of benzene, toluene, ethylbenzene and xylene.

3. The process according to Claim 1, wherein the $C_2$-$C_4$ olefin is at least one member selected from the group consisting of ethylene, propylene, butene-1, trans-butene-2 and cis-butene-2.

4. The process according to Claim 1, wherein the aromatic hydrocarbon is benzene and the $C_2$-$C_4$ olefin is ethylene.

5. The process according to Claim 1, wherein the $\beta$-zeolite is an acid type $\beta$-zeolite in which the $SiO_2/Al_2O_3$ ratio is in the range of 5 to 100 and sodium ions are substituted with hydrogen ions or polyvalent cations.

**Patentansprüche**

1. Verfahren zur Alkylierung von aromatischen Kohlenwasserstoffen in flüssiger Phase, welches das Umsetzen eines aromatischen Kohlenwasserstoffs mit einem $C_2$-$C_4$-Olefin in einer flüssigen Phase in einem Rieselströmungsbereich in einem Festbettreaktor, der mit einem $\beta$-Zeolithkatalysator gepackt ist, entsprechend einem Reaktionssystem mit abwärts gerichtetem Gleichstrom bei 100 bis 300°C umfaßt, wobei der Rieselströmungsbereich durch die folgenden Bedingungen spezifiziert ist:

$$\rho_l \cdot u_l \cdot \{(\sigma_{Wasser}/\sigma) \ (\rho_{Wasser}/\rho_l)^2\}^{1/3}(kgm^{-2}s^{-1}) \leq 10$$

$$\rho_g \cdot u_g \cdot \{\rho_{Luft} \cdot \rho_{Wasser}/(\rho_g \cdot \rho_l)\}^{\frac{1}{2}}(kgm^{-2}s^{-1} \leq 1$$

worin $\rho_l$, $\rho_g$, $\rho_{Luft}$ und $\rho_{Wasser}$ ($kgm^{-3}$) die Flüssigkeitsdichte des aromatischen Kohlenwasserstoffs, die Gasdichte des $C_2$-$C_4$-Olefins, die Gasdichte von Luft bzw. die Flüssigkeitsdichte von Wasser bezeichnen, $\sigma$ und $\sigma_{Wasser}$ ($Nm^{-1}$) die Oberflächenspannung des aromatischen Kohlenwasserstoffs bzw. von Wasser bezeichnen und $ul$ und $ug$ ($ms^{-1}$) die Oberflächengeschwindigkeit des aromatischen Kohlenwasserstoffs bzw. $C_2$-$C_4$-Olefins in der Säule bezeichnen.

2. Verfahren nach Anspruch 1, wobei der aromatische Kohlenwasserstoff mindestens ein aus der aus Benzol , Toluol, Ethylbenzol und Xylol bestehenden Gruppe ausgewähltes Mitglied ist.

3. Verfahren nach Anspruch 1, wobei das $C_2$-$C_4$-Olefin mindestens ein aus der aus Ethylen, Propylen, Buten-1, trans-Buten-2 und cis-Buten-2 bestehenden Gruppe ausgewähltes Mitglied ist.

4. Verfahren nach Anspruch 1, wobei der aromatische Kohlenwasserstoff Benzol und das $C_2$-$C_4$-Olefin Ethylen ist.

5. Verfahren nach Anspruch 1, wobei der $\beta$-Zeolith ein $\beta$-Zeolith vom Säuretyp ist, in dem das $SiO_2/Al_2O_3$-Verhältnis im Bereich von 5 bis 100 ist und Natriumionen durch Wasserstoffionen oder mehrwertige Kationen ersetzt sind.

**Revendications**

1. Procédé d'alkylation en phase liquide d'hydrocarbures aromatiques, qui consiste à mettre un hydrocarbure aromatique à réagir sur une oléfine ayant de 2 à 4 atomes de carbone en phase liquide dans une région d'écoulement goutte à goutte dans un réacteur à lit fixe garni de catalyseur à base de zéolite β suivant un système réactionnel à courant descendant et de même sens entre 100 et 300°C, la région d'écoulement goutte à goutte étant spécifiée en ce qu'elle satisfait aux conditions suivantes :

$$\rho l.ul.\{(\sigma water/\sigma)(\rho water/\rho l)^2\}^{1/3}\ (kgm^{-2}s^{-1}) \leq 10$$

$$\rho g.ug.\{\rho air.\rho water/(\rho g.\rho l)\}^{\frac{1}{2}}\ (kgm^{-2}s^{-1}) \leq 1$$

dans lesquelles $\rho l$, $\rho g$, $\rho air$ et $\rho water$ ($kgm^{-3}$) dénotent la masse volumique du liquide de l'hydrocarbure aromatique, la masse volumique gazeuse de l'oléfine ayant de 2 à 4 atomes de carbone, la masse volumique gazeuse de l'air et la masse volumique liquide de l'eau, respectivement, $\sigma$ et $\sigma water$ ($Nm^{-1}$) dénotent les tensions superficielles de l'hydrocarbure aromatique et de l'eau, respectivement, et $ul$ et $ug$ ($ms^{-1}$) dénotent les vitesses superficielles dans une colonne de l'hydrocarbure aromatique et de l'oléfine ayant de 2 à 4 atomes de carbone, respectivement.

2. Procédé suivant la revendication 1, dans lequel l'hydrocarbure aromatique est au moins un membre choisi dans le groupe consistant en le benzène, le toluène, l'éthylbenzène et le xylène.

3. Procédé suivant la revendication 1, dans lequel l'oléfine ayant de 2 à 4 atomes de carbone est au moins un membre choisi dans le groupe consistant en l'éthylène, le propylène, le butène-1, le trans-butène-2 et le cis-butène-2.

4. Procédé suivant la revendication 1, dans lequel l'hydrocarbure aromatique est le benzène et l'oléfine ayant de 2 à 4 atomes de carbone est l'éthylène.

5. Procédé suivant la revendication 1, dans lequel la zéolite β est une zéolite β de type acide dans laquelle le rapport $SiO_2/Al_2O_3$ est de l'ordre de 5 à 100 et des ions sodium sont remplacés par des ions hydrogène ou des cations polyvalents.

# F IG. 1

SPRAY FLOW

PULSED FLOW

TRICKLE FLOW

BUBBLE FLOW

$\rho g \cdot ug \{\rho air \cdot \rho water / (\rho g \cdot \rho l)\}^{1/2} (kgm^{-2}s^{-1})$

$\rho l \cdot ul \{(\sigma water / \sigma)(\rho water / \rho l)^2\}^{1/3} (kgm^{-2}s^{-1})$